# EUROPEAN PATENT SPECIFICATION

(11) **EP 1 757 349 B1**
(45) Date of publication and mention of the grant of the patent: **21.10.2009**
(21) Application number: 06002849.5
(22) Date of filing: 13.02.2006
(51) Int. Cl.: B01D 15/38, G01N 33/538, G01N 33/545

(54) **Multi-analyte affinity column for mycotoxins**
Multianalyt-Affinitätssäule für Mykotoxine
Colonne par affinité multi-analytes pour mycotoxines

(30) Priority: 26.08.2005 US 711524 P; 18.11.2005 US 283159
(43) Date of publication of application: 28.02.2007
(73) Proprietor: Waters Investments Limited, New Castle, Delaware 19720 (US)
(72) Inventor: Zabe, Nancy Ann, Waltham MA 02453 (US); Basker, Christopher John, Worcester MA 01606 (US)
(74) Representative: Oser, Andreas

(56) References cited:
- US-A1- 2005 100 959
- OFITSEROVA M ET AL: "Multi-Residue Mycotoxin Analysis"[Online] XP002377222 Retrieved from the Internet: URL:http://www.pickeringlabs.com.cn/suppor t/appnotes/MA203.pdf>
- GOEBEL R ET AL: "SIMULTANEOUS DETERMINATION OF AFLATOXINS, OCHRATOXIN A, AND ZEARALENONE IN GRAINS BY NEW IMMUNOAFFINITY COLUMN LIQUID CHROMATOGRAPHY" JOURNAL OF AOAC INTERNATIONAL, AOAC INTERNATIONAL, ARLINGTON, VA, US, vol. 87, no. 2, 2004, pages 411-416, XP008061200 ISSN: 1060-3271
- SCOTT P M ET AL: "APPLICATION OF IMMUNOAFFINITY COLUMNS TO MYCOTOXIN ANALYSIS" JOURNAL OF AOAC INTERNATIONAL, AOAC INTERNATIONAL, ARLINGTON, VA, US, vol. 80, no. 5, September 1997 (1997-09), pages 941-949, XP008061305 ISSN: 1060-3271
- DATABASE BIOSIS [Online] BIOSCIENCES INFORMATION SERVICE, PHILADELPHIA, PA, US; June 2003 (2003-06), VAN DER GAAG BRAM ET AL: "Biosensors and multiple mycotoxin analysis." XP002377575 Database accession no. PREV200300291091 & FOOD CONTROL, vol. 14, no. 4, June 2003 (2003-06), pages 251-254, ISSN: 0956-7135

## Description

### FIELD OF THE INVENTION

The invention is concerned with affinity columns used for immunological screening for environmentally occurring toxins, for example, those found in food products, and is particularly directed to multi-analyte columns for detecting a plurality of toxins that may be present in a single sample.

### BACKGROUND OF THE INVENTION

Awareness of the incidence and effect of human and animal exposure to toxic substances by humans and other animals via food, water, and air is of critical importance to our survival. The detection of toxins such as aflatoxin, ochratoxin, zearalenone, deoxynivalenol and fumonisin has become especially important. In particular, screening procedures for assessing the exposure of humans to such toxins may require the ability to quantify both the toxin and its metabolites.

Aflatoxins are a typical example of the compounds for which screening is desired. Aflatoxins are secondary fungal metabolites, mycotoxins, which are produced by *Aspergillus flavus* and *Aspergillus parasiticus* and are structurally a group of substituted coumarins containing a fused dihydrofurofuran moiety. Aflatoxins occur naturally in peanuts, peanut meal, cottonseed meal, corn, dried chili peppers, and the like. However, the growth of the mold itself does not predict the presence or levels of the toxin because the yield of aflatoxin depends on growth conditions as well as the genetic requirements of the species. A variety of aflatoxins, that is types B₁, B₂, G₁, G₂, M₁ and M₂, have been isolated and characterized. Aflatoxin B₁ ("AFB₁") is the most biologically potent of these aflatoxins and has been shown to be toxic, mutagenic and carcinogenic in many animal species. This mycotoxin is a frequent contaminant of the human food supply in many areas of the world and is statistically associated with increased incidence of human liver cancer in Asia and Africa, in particular (Busby et al., in Food-Born Infections and Intoxications (Riemann and Bryan, Editors) Second Edition, Academic Press, Inc., 1979, pp. 519-610; Wogan, G. N. Methods Cancer Res.7:309-344 (1973)).

AFB₁ also forms covalently linked adducts with guanine in DNA after oxidative metabolism to a highly reactive 2,3-exo-epoxide, the major adduct product being 2,3-dihydro-2-(N₇-guanyl)-3-hydroxy-aflatoxin B₁ ("AFB₁-N7-Gua") (Lin et al., Cancer Res. 37:4430-4438 (1977); Essigman et al., Proc. Natl. Acad. Sci. USA 74:1870-1874 (1977); Martin et al., Nature (London) 267:863-865 (1977)). The AFB₁-N7-Gua adduct and its putative derivatives (2,3-dihydro-2-(N5-formyl-2',5',6'-triamino-4'-oxo'N5-pyrimidyl)-3-hydroxy-aflatoxin B₁) ("AF-N7-Gua") have been identified in a wide variety of tissues and systems such as rat liver in vivo, cultured human bronchus and colon, and human lung cells in culture after acute or chronic administration (Haugen et al., Proc. Natl. Acad. Sci. USA 78:4124-4127 (1981)).

Some investigations regarding quantitation of aflatoxin B₁ and its metabolites including its DNA adduct have been conducted using immunological techniques and monoclonal antibodies (Hertzog et al., Carcinogensis 3:825-828 (1982); Groopman et al., Cancer Res. 42:3120-3124 (1982); Haugen et al., Proc. Natl. Acad. Sci. USA 78: 4124-4127 (1981)). Similar research has been conducted utilizing immunological techniques and reagents for other low molecular weight toxins found in our environment (Johnson et al., J Analyt. Toxicol. 4:86-90 (1980); Sizaret et al., J.N.C.I. 69:1375-1381 ( 1982); Hu et al., J. Food Prot. 47:126-127. (1984); and Chu, J. Food Prot. 47:562-569 (1984)).

U.S. Patent No. 4,818,687 describes a general non-invasive screening procedure for assessing the exposure of humans and animals to environmentally occurring carcinogens. Therein, an affinity matrix and a method for the detection of low molecular weight compositions such as aflatoxins are provided utilizing specific monoclonal IgM antibody.

R. Göbel et. al., "Simultaneous determination of aflatoxins, ochratoxin A, and zearalenone in grains by new immunoaffinity column/liquid chromatography", Journal of AOAC International, Vol. 87, No. 2, 2004, p. 411-416 describes an immunoaffinity column capable of analyzing aflatoxine, ochratoxine and zearalenone simultaneously.

P. M. Scott et. al., "Application of immunoaffinity columns to mycotoxin analysis", Journal of AOAC International, Vol. 80, No. 5, 1997, p. 941-949 is a review concerning, among others, the reuse of immunoaffinity columns in mycotoxin analysis.

The US 2005/0100959 A1 discloses a device and method for detecting the presence of an analyte, among others toxins.

Affinity columns for detecting the presence of a single analyte, for example, one of aflatoxin, ochratoxin, zearalenone, deoxynivalenol or fumonisin, in a sample are well known. An affinity column for detecting both aflatoxin and ochratoxin in a single sample as well as an affinity column for detecting aflatoxin, ochratoxin and zearalenone have been commercially available. However, columns targeting higher numbers of chemical species necessarily must capture more diverse analytes. Aflatoxin is a large aromatic, multi-ring structure. Deoxynivalenol (DON) is a highly polar toxin that is smaller than a molecule of table sugarsucrose. The lipid-like fumonisin shares structural characteristics with sphingolipids. Thus, the preparation of multi-analyte columns and their methods of use increase in complexity far out of proportion to the number of toxins being added for analysis. Column development must allow for treatment of all target analytes according to similar methods, in order that they all be analyzed with a single column.

There have been numerous reported incidences of naturally-occurring mycotoxins such as, aflatoxin B₁, B₂, G₁, G₂ and M₁ (Afla), deoxynivalenol (DON), fumonisin B₁, B₂ and B₃, ochratoxin A (OTA), and zearalenone (Zear) in various substrates. Malt beverages and wines can contain different multi-toxin combinations from fungi-infected grains and fruits used in the production. A desire still exists for competent multi-analyte columns for analyzing a plurality of toxins with a single column.

### SUMMARY OF THE INVENTION

The present invention provides a multi-analyte column capable of analyzing a single sample containing one or more of aflatoxin, deoxynivalenol ("DON"), fumonisin, ochratoxin and zearalenone. The muti-analyte columns in accord with the present invention comprise a first quantity of a first resin comprising an antibody having specificity for aflatoxin, a second quantity of a second resin comprising an antibody having specificity for deoxynivalenol, a third quantity of a third resin comprising an antibody having specificity for fumonisin, a fourth quantity of a fourth resin comprising an antibody having specificity for ochratoxin and a fifth quantity of a fifth resin comprising an antibody having specificity for zearalenone.

It is desirable to obtain at least a 60%, preferably at least a 70% recovery from the column for each toxin in the sample. It also is desirable to have a column flow rate of at least 3 ml per minute, preferably so that a 10 ml sample will flow through the column in less than 5min. We have found that it is not possible to obtain satisfactory analytical results in a multi-analyte column by merely combining the quantities of resin used in a single analyte column to analyze each particular analyte.

Thus, we have found that a multi-analyte column capable of analyzing a single sample containing aflatoxin, deoxynivalenol, fumonisin, ochratoxin and zearalenone, comprises for each unit of resin containing antibody having specificity for ochratoxin, about 0.95 to 1.05 units of resin containing antibody having specificity for zearalenone, about 1.9 to 2.1 units of resin containing antibody having specificity for aflatoxin, about 2.35 to 2.65 units of resin containing antibody having specificity for fumonisin and about 4.7 to 5.3 units of resin containing antibody having specificity for deoxynivalenol. As used herein, one unit of resin is defined as the quantity of resin containing antibody that will bind 50 ng of aflatoxin, 500 ng of deoxynivalenol, 3330 ng of fumonisin, 50 ng of ochratoxin or 1140 ng of zearalenone, respectively. Such resin typically will contain about 5 mg antibody per ml of resin. However, any suitable loading of antibody on the resin can be used in accord with quantities and methods well known to those skilled in the art.

In a preferred embodiment, the multi-analyte column of the present invention is capable of analyzing a sample to detect aflatoxins G₁, G₂, B₁, B₂ and M₁, DON, fumonisins B₁, B₂ and B₃, ochratoxin A and zearalenone in the analysis of a single sample applied to the column.

The invention also provides a method for analyzing a single sample for aflatoxin, deoxynivalenol, fumonisin, ochratoxin and zearalenone, the method comprising providing a multi-analyte column as described herein, applying liquid sample suspected of containing one or more of the specified toxins to bind any of the specified toxins to resins in the column, washing the column, eluting the resins and analyzing the eluant for the presence of each of the specified toxins. The liquid sample can be a liquid suspected of containing toxins or a liquid extract of a solid material suspected of containing toxins. Specific examples of sample materials that can be analyzed in accord with the columns of the present invention include fungi-infected grains and fruits, and alcoholic beverages such as, for example, malt beverages and wines.

### DETAILED DESCRIPTION OF THE INVENTION INCLUDING PREFERRED EMBODIMENTS

In accord with the present invention, a multi-analyte column capable of analyzing a single sample containing aflatoxin, deoxynivalenol, fumonisin, ochratoxin and zearalenone can be prepared. Resins containing antibody having specificity for each of the toxins are required. Antibodies are raised by well known techniques and monoclonal antibodies are prepared having specificity for each toxin. Resins having each antibody bound thereto are prepared by techniques well known to those skilled in the art. Any resin material known by those skilled in the art to be useful for carrying attached antibodies can be used. A preferred resin material is Sepahrose® 4B available from Amersham Biosciences (Piscataway, NJ). The antibodies are then attached to the resin using techniques well known to those skilled in the art. Preferably, about 5 mg of antibody is bound to one ml of resin. The resin preferably has a particle size range of about 45 to about 165 µm.

Columns are then prepared using appropriate quantities of each resin. For example, in one embodiment of the invention, in a 3 ml column having a diameter of 8.93 mm, a supporting porous disk, or the like, is positioned to support the resin bed while permitting flow out of the column. 200 µl of a first resin having an antibody specific for aflatoxin is layered on the disk. Then, 100 µl of a second resin having an antibody specific for ochratoxin is layered on the first resin. Then, 250 µl of a third resin having an antibody specific for fumonisin is layered on the second resin. Then, 100 µl of a fourth resin having an antibody specific for zearalenone is layered on the third resin. 500 µl of a fifth resin having an antibody specific for DON is layered on the fourth resin. Finally, another porous disk, or the like, if desired, can be positioned to distribute the liquid sample across the column and/or hold the resin in place. Alternatively, the resins can be mixed together and then loaded into the column as a mixture. Further, a suitable porous media such as, e.g., glass wool or the like, can be used in place of the porous disk.

For comparable size single analyte columns performing the same task, the same antibody / resins typically are loaded presently at 250 µl for aflatoxin, 250 µl for ochratoxin, 350 µl for fumonisin, 350 µl for zearalenone and 550 µl for DON.

In the above embodiment, 100 µl of resin is equal to one unit. Each unit of resin is capable of binding about 50 ng of aflatoxin, 500 ng of deoxynivalenol, 3300 ng of fumonisin, 50 ng of ochratoxin or 1140 ng of zearalenone, respectively. In accord with the invention, for each unit of resin having ochratoxin specific affinity, the column contains about 0.95 to 1.05 units of resin containing antibody having specificity for zearalenone, about 1.9 to 2.1 units of resin containing antibody having specificity for aflatoxin, about 2.35 to 2.65 units of resin containing antibody having specificity for fumonisin and about 4.7 to 5.3 units of resin containing antibody having specificity for deoxynivalenol.

The total amount of resin in the column should permit a sample fluid to flow through the column at a preferred rate of about 1-2 drops per sec.

For solid foods, preferably toxins are extracted from the food using a water-based or water compatible solvent such as, for example, water:methanol, water:acetonitrile, ethanol, water:ethanol, salt solutions, buffer solutions, and the like, etc. Such solvents are well known to those skilled in the art. Typically, in such solvents the organic component is greater. Extracts can be diluted with water prior to chromatography.

After loading the sample on the column, the column typically is washed to remove any extraneous materials that may be held up on the column so that only bound materials, i.e., the toxins, remain. The column generally can be washed with the water compatible solvent but typically having a greater water presence.

The column is eluted with solvents as is well known to those skilled in the art. The eluants are analyzed for the particular analytes using HPLC techniques equipped with in-line photochemical reactor, post column derivatizer, ultraviolet and fluorescent detectors.

Muti-analyte columns in accord with the present invention can be used as a clean-up step in analysis of extracts from solid materials or of liquid products such as alcoholic beverages for aflatoxins, fumonisins, ochratoxin A, deoxynivalenol and zearalenone, in combination with HPLC and/or mass spectrometry detection. The detection of the toxin can be illustrated, typically, by spiking a sample of a solid, extract, malt beverage or rice wine with toxins. If desired, the sample can be dried to eliminate the alcohol content. Then resuspend the dried sample in deoionized water or phosphate buffered saline (PBS) to a volume equal to the original sample. Dilute the resuspended sample 1.1 (v/v) in 1/10 diluted phosphate buffered saline 10x stock solution from VICAM (pH of sake and beer samples are roughly between 5.0 and 6.0). Load the sample onto the multi-analyte column at a speed of about 2 drops/second. Wash the column with deonized water or phosphate buffered saline). Elute the toxins from the column with methanol. Dry the aqueous methanolic eluate and reconstitute in methanol. Inject about a 30u1 sample onto the HPLC. Preferably, the HPLC is equipped with in-line photochemical reactor (PHRED), post-column derivatizer, ultra-violet and fluorescence detectors. Aflatoxins are detected by fluoresence after post-column photochemical derivitization (post-column iodine may also be used). Fumonisin is derivatized with o-phthaldialdehyde and detected by fluoresence. DON is detected by UV absorbance. Zearalenone is detected by fluoresence. Ochratoxin is detected by fluoresence. Methods for detecting the toxins are well known to those skilled in the art.

Alcoholic beverages can contain naturally occurring multiple mycotoxins. A single sample of an alcoholic beverage can be analyzed for aflatoxin, deoxynivalenol, fumonisin, ochratoxin and zearalenone using the five analyte column of the present invention.

The following example illustrates detection of aflatoxins G₁, G₂, B₁, B₂, DON, fumonisins B₁, B₂, B₃, ochratoxin A and zearalenone using a column containing 200 µl of a first resin having an antibody specific for aflatoxin, 100 µl of a second resin having an antibody specific for ochratoxin, 250 µl of a third resin having an antibody specific for fumonisin, 100 µl of a fourth resin having an antibody specific for zearalenone and 500 µl of a fifth resin having an antibody specific for DON, wherein each resin has 5 mg/ml of antibody and toxin detection capability per unit described herein. Spiked samples were used to calculate recovery from the column.

### Materials and Methods

### Reagents and Chemicals

The o-phthaldialdehyde (OPA) solid, OPA diluent (5.4% potassium borate in water), Thiofluor^{™} (N,N-Dimethyl-2-mercaptoethylamine hydrochloride) solid, and phosphoric buffer solution (P/N 1700-1108) were received from Pickering Laboratories (Mountain View, CA). The 30% (w/v) Brij® 35 solution was obtained from Sigma (St. Louis, MO). Acetonitrile and methanol (both Optima grade) were obtained from Fisher Scientific (Pittsburgh, PA). Deionized water was produced by a Millipore Milli-Q system (Bedford, MA). Amber glass ampules of aflatoxin B₁, B₂, G₁ & G₂, deoxynivalenol, ochratoxin A, and zearalenone standards in appropriate organic solvents were obtained from Supelco (Bellefonte, PA). Fumonisin B₁, B₂ & B₃ were donated by PROMEC of Medical Research Council (Tygersberg, South Africa). SurfaSil™ siliconizing fluid for surface treatment of in-house laboratory glassware was obtained from Pierce Biotechnology (Rockford, IL).

### Reagent Preparation

OPA reagent preparation: One 950-mL size bottle containing the OPA diluent was sparged with helium (inert gas) for 10 min. A 300-mg OPA portion was added into 50 mL beaker and dissolved in 10 mL methanol. A 2-g Thiofluor^{™} portion was then added into 50 mL beaker. The inert gas was turned off and the OPA solution and Thiofluor^{™} mixture were added to the sparged diluent. A 3-mL of 30% (w/v) Brij^{®} 35 solution was also added and then mixed well with the rest. This specially prepared reagent is readily oxidized and should be kept under inert gas. Saran^{™} (polyvinylidene chloride) tubing for inert gas supply and reagent connections was used to minimize this problem.

Multi-toxin stock standard solution preparation: Accurately measured amounts of all five mycotoxin families were transferred into a silanized borosilicate glass volumetric flask. Accompanying organic solvents were dried and reconstituted with deionized water, and filled to the mark to prepare a known mixed-toxin stock standard solution to be used for multi-toxin standard calibration and sample spiking purposes.

### Apparatus and Equipment

The complete system apparatus contained several instruments that were assembled in series (HPLC injector ― analytical column ― ultra-violet (UV) detector ― photochemical reactor ― post-column derivatizer ― fluorescence detector ― waste). The HPLC set-up consisted of Agilent 1100 Series quaternary pump and injection system, including a standard autosampler. The 1100 Series fluorescence and diode-array detector (DAD) from Agilent Technologies (Palo Alto, CA) were used.

### Analytical Conditions

The MycoTox™, C₁₈ analytical column, 4.6x250 mm, 5 µm particle size, and a 5-µm guard column were from Pickering Laboratories (Mountain View, CA). Agilent's ChemStation software was used for data management. The mobile phase consisted of combinations of three reagents. The HPLC gradient was as follows:

**Table 1: HPLC gradient**

| **Time** | **Phosphoric buffer (P/N 1700-1108), %** | **Methanol, %** | **Acetonitrile, %** |
|---|---|---|---|
| 0.0 | 85 | 0 | 15 |
| 5.0 | 85 | 0 | 15 |
| 5.1 | 57 | 28 | 15 |
| 20.0 | 57 | 28 | 15 |
| 23.0 | 40 | 60 | 0 |
| 40.0 | 40 | 60 | 0 |
| 50.0 | 0 | 100 | 0 |
| 60.0 | 0 | 100 | 0 |

The flow rate was 1 mL/min with column temperature of 40° C and injection volume of 30 µL. The equilibration time was 10 min.

### Photochemical Reactor for Enhanced Detection ("PHRED"™)

The PHRED^{™} unit (Aura Industries, New York, NY) was equipped with a 254 nm low pressure Hg lamp and the PTFE (poly-tetrafluoroethylene) knitted reactor coils. The 254-nm UV light was able to perform continuous photolytic derivatization to enhance the sensitivity and/or selectivity of fluorescence detection response. The photochemical reactor was placed between the HPLC analytical column and the detector.

**Table 2: Detection**

| Analyte | Derivatization | Detection | Wavelength |
|---|---|---|---|
| DON | None | Ultra-violet | λ=218 nm |
| Aflatoxins | Photolytic (PHRED^{™}) | Fluorescence | λₑₓ = 365 nm |
| | | | λₑₘ = 455 nm |
| Fumonisins | Post-column (OPA) | Fluorescence | λₑₓ = 330 nm |
| | | | λₑₘ = 465 nm |
| Ochratoxin A | None | Fluorescence | λₑₓ = 335 nm |
| | | | λₑₘ = 455 nm |
| Zearalenone | None | Fluorescence | λₑₓ = 275 nm |
| | | | λₑₘ = 455 nm |

**Table 3: The wavelength settings on the fluorescence detector were as follows:**

| **Time** | **λₑₓ** | **λₑₘ** |
|---|---|---|
| 0.0 | 365 | 455 |
| 26.0 | 365 | 455 |
| 26.1 | 330 | 465 |
| 36.0 | 330 | 465 |
| 36.1 | 335 | 455 |
| 41.0 | 335 | 455 |
| 41.1 | 275 | 455 |
| 44.0 | 275 | 455 |
| 44.1 | 330 | 465 |
| 60.0 | 330 | 465 |

The highest detector sensitivity level at PMT gain 16 was selected. All gradient and wavelength changes were programmed through the ChemStation software.

### Post-column Conditions

The PCX5200 post-column derivatization system was equipped with Control Software from Pickering Laboratories (Mountain View, CA). The reactor volume and temperature were set at 1.4 ml and 65°C, respectively. The derivatizing reagent was a specially prepared OPA reagent. The flow rate was set at 0.3 ml/min. The post-column pump program was activated using the PCX5200 Control Software, which turned the pump on at 23.0 min, then off at 34.5 min and on again at 43.5 min and finally off at 60.0 min.

### Sample Preparation and SPE Column Clean-up Protocols

The Visiprep^{®} 24-port SPE vacuum manifold and Visidry^{®} drying attachment from Supelco (Bellefonte, PA) or the RapidTrace^{®} automated SPE workstation from Zymark/Caliper LifeSciences (Hopkinton, MA) were used for sample preparations. In this example, the alcoholic beverage sample was dried to remove alcohol and other volatile organic constituents, and then reconstituted to its original volume in one-tenth diluted PBS solution. Either a 5-ml aliquot of alcoholic beverage in one-tenth diluted PBS solution spiked with multi-toxin standards (sample) or a one-tenth diluted PBS solution spiked with multi-toxin standards (control) was passed through the multi-toxin antibody-based SPE column. Larger sample volumes can be added if a mycotoxin pre-concentration step is desired. The IA column was washed with 4 ml deionized water. Target mycotoxins were eluted with 3 ml methanol. The water-washing step was done at a flow rate of about 2 drops/sec, but the sample loading and methanol-elution steps were performed at a slower rate (≤ 1 drop/sec). Then, the methanol eluate collected in a silanized borosilicate culture tube was dried and reconstituted in 3 ml methanol. The methanol eluate was either dried down at ambient temperature using air or at 40°C under nitrogen. During the enrichment step the dried mycotoxins were then reconstituted with a smaller amount of methanol compared to the original sample volume loaded on to the column. The process was done in a silanized borosilicate tube tightly covered by a piece of Parafilm^{®} film or a plastic cap, and mixed well using the Vortex-Genie™ vortexer from Scientific Industries (Bohemia, NY). Thirty microliters of the prepared sample solution was injected into the HPLC.

This HPLC method simultaneously analyzes aflatoxins, DON, fumonisins, ochratoxin A and zearalenone with post-column photochemical and o-phthaldialdehyde (OPA) derivatizations. The ruggedness of separation and detection has been established on a representative multi-toxin mid-level calibration standard chromatogram. Generated 5-point multi-toxin standard calibration curves showed linear regression correlation coefficients ≥ 0.999.

The ultra-violet detector and photochemical reactor were strategically placed in series before the post-column derivatizer hardware for the simultaneous UV detection of DON and photolytic derivatization of aflatoxins. The method allows for fluorescent detection of the fumonisins using the prepared OPA reagent, aflatoxins via photolysis and the natural-fluorescence of zearalenone and ochratoxin A. Fumonisins have a primary amine group that is derivatized post-column with OPA and a mercaptan to form a highly-fluorescent adduct, 1-alkyl-2-thioalkyl-subsitituted isoindole exhibiting optimal excitation at 330 nm and maximal emission at 465 nm. The OPA reagent flow was started after the aflatoxins elution and stopped after the fumonisin B₁ peak elution. Sufficient delay time was allocated to flush the OPA from the tubing prior to the ochratoxin A and zearalenone elution. Then, the OPA reagent flow was turned back on during the fumonisin B₃ and B₂ elution. The fluorescence detector was time-programmed to change excitation and emission wavelengths for multi-toxin response optimization.

The multi-toxin recoveries in spiked PBS control and alcoholic beverage samples with the enrichment step in the silanized borosilicate tube ranged from 70.9 to 110.6 % with RSD ≤ 10% in nearly all the data at n = 3 (Table 4). The acceptable multi-toxin spike recovery ranges demonstrated the 5 analyte IA column's ability to effectively and selectively bind with the targeted mycotoxins.

**Table 4: Spike and Recoveries (%) Using the 5-toxin (AflaDONFumoOTAZear; "AFOZD") Antibody-based SPE Column Clean-up Experiment Using the Negative-pressure SPE Manifold**

| | 5-toxin Method: IA Column (n=3) Spiked PBS (Control) | 5-toxin Method: IA Column (n=3) Spiked Light Beer | 5-toxin Method: IA Column (n=3) Spiked Rice Wine^{#} |
|---|---|---|---|
| DON | 104.6 ± 4.5 | 93.8 ± 4.1 | 95.2 ± 3.3 |
| Afla G₂ | 78.9 ± 8.3 | 74.9 ± 12.1 | 93.7 ± 2.7 |
| Afla G₁ | 70.9 ± 9.2 | 72.9 ± 13.3 | 91.3 ± 3.8 |
| Afla B₂ | 112.0 ± 7.2 | 88.1 ± 7.7 | 98.8 ± 1.5 |
| Afla B₁ | 106.8 ± 5.5 | 93.0 ± 9.0 | 96.4 ± 1.7 |
| Fumo B₁ | 83.0 ± 7.7 | 76.9 ± 2.8 | 83.9 ± 12.6 |
| Fumo B₂ | 100.1 ± 12.4 | 98.6 ± 7.8 | 82.5 ± 4.2 |
| Fumo B₃ | 90.1 ± 10.7 | 92.9 ± 1.5 | 83.5 ± 7.8 |
| OTA | 95.7 ± 1.6 | 101.8 ± 6.9 | 89.2 ± 4.3 |
| Zear | 88.1 ± 3.2 | 100.8 ± 7.4 | 110.6 ± 6.4 |

| | | | |
|---|---|---|---|
| ^{#} Cloudy Rice Wine with cooked rice particulate matter. Averaged mycotoxin recoveries in percent (%) ± standard deviation (SD). Majority data at n = 3 with relative standard deviation (RSD) ≤ 10 %. | | | |

The present inventions have been described in detail including preferred embodiments thereof. However, it should be appreciated that those skilled in the art, upon consideration of the present disclosure, may make modifications and improvements.

## Claims

1. A multi-analyte column comprising at least one unit of resin having ochratoxin specific affinity and, for each unit of resin having ochratoxin specific affinity, the column further contains 0.95 to 1.05 units of resin containing antibody having specificity for zearalenone, 1.9 to 2.1 units of resin containing antibody having specificity for aflatoxin, 2.35 to 2.65 units of resin containing antibody having specificity for fumonisin and 4.7 to 5.3 units of resin containing antibody having specificity for deoxynivalenol, wherein one unit of resin is the quantity of resin containing antibody that will bind 50 ng of aflatoxin, 500 ng of deoxynivalenol, 3300 ng of fumonisin, 50 ng of ochratoxin or 1140 ng of zearalenone, respectively.

2. The multi-analyte column of claim 1, wherein the column is structured and arranged to recover at least 60%, preferably at least 70% of a toxin in a 10 ml sample being analyzed, said toxin being selected from the group consisting of ochratoxin, zearalenone, aflatoxin, fumonisin and deoxynivalenol.

3. The mult'i-analyte column of claim 1 or 2, wherein the column is structured and arranged to have a column flow rate of at least 3 ml per minute, or
wherein a flow rate of sample fluid through the column is 1-2 drops per second.

4. The multi-analyte column of any one of claims 1 to 3, wherein the column is capable of analyzing a sample to detect each of aflatoxins G₁, G₂, B₁, B₂ and M₁, deoxynivalenol "DON", fumonisins B₁, B₂ and B₃, ochratoxin A and zearalenone.

5. The multi-analyte column of any one of claims 1 to 4, wherein the resin has a particle size of 45 to 165 µm, and / or
wherein the resin comprises 5 mg of antibody per ml of resin.

6. The multi-analyte column of any one of claims 1 to 5, wherein one resin having a toxin specific affinity is layered into a column followed successively by layering into the column another resin having a different toxin specific affinity until all of the resin is in the column, or
wherein the resins having different toxin specific affinity are mixed and placed into a column.

7. A method of analyzing a single liquid sample for aflatoxin, deoxynivalenol, fumonisin, ochratoxin and zearalenone, the method comprising:
providing a multi-analyte column as set forth in any of claims 1 to 6;
loading the column with a predetermined amount of a liquid sample suspected of containing one or more of the toxins selected from aflatoxin, deoxynivalenol, fumonisin, ochratoxin and zearalenone;
binding the toxins to the antibodies on the column;
subsequently, eluting each of the toxins in eluant; and
analyzing the eluant for the presence of each toxin.

8. The method according to claim 7, wherein toxins are extracted from a food using a water-based or water compatible solvent.

9. The method according to claim 8, wherein the solvent is water:methanol, water:acetonitrile, ethanol, water:ethanol, a salt solution or a buffer solution.

10. The method according to claim 7, wherein the liquid sample comprises any one of the group consisting of a food extract, a grain extract, and an alcoholic beverage.

11. A use of a multi-analyte column as set forth in any of claims 1 to 6 for analyzing a food product or component, for analyzing an alcoholic beverage, for analyzing fungi-infected grains and fruits, or for analyzing malt beverages and wines.

## Patentansprüche

1. Eine Multi-Analyt-Säule, die mindestens eine Ochratoxin-spezifische Affinität aufweisende Harzeinheit umfasst, und für jede Ochratoxin-spezifische Affinität aufweisende Harzeinheit enthält die Säule ferner 0,95 bis 1,05 Harzeinheiten mit Spezifität bezüglich Zearalenon aufweisendem Antikörper, 1,9 bis 2,1 Harzeinheiten mit Spezifität bezüglich Aflatoxin aufweisendem Antikörper, 2,35 bis 2,65 Harzeinheiten mit Spezifität bezüglich Fumonisin aufweisendem Antikörper, und 4,7 bis 5,3 Harzeinheiten mit Spezifität bezüglich Deoxynivalenol aufweisendem Antikörper, wobei eine Harzeinheit die Menge an Antikörper enthaltendem Harz ist, welche jeweils 50 ng Aflatoxin, 500 ng Deoxynivalenol, 3300 ng Fumonisin, 50 ng Ochratoxin oder 1140 ng Zearalenon bindet.

2. Die Multi-Analyt-Säule von Anspruch 1, wobei die Säule aufgebaut und angeordnet ist, um mindestens 60%, bevorzugt mindestens 70% eines Toxins in einer 10 ml Probe, die analysiert wird, zurückzugewinnen, wobei das Toxin aus der aus Ochratoxin, Zearalenon, Aflatoxin, Fumonisin und Deoxynivalenol bestehenden Gruppe ausgewählt wird.

3. Die Multi-Analyt-Säule von Anspruch 1 oder 2, wobei die Säule aufgebaut und angeordnet ist, um eine Säulenfließgeschwindigkeit von mindestens 3 ml pro Minute aufzuweisen, oder
wobei eine Fließgeschwindigkeit von Probenfluid durch die Säule 1-2 Tropfen pro Sekunde ist.

4. Die Multi-Analyt-Säule von irgendeinem der Ansprüche 1 bis 3, wobei die Säule imstande ist, eine Probe zu analysieren, um jedes der Aflatoxine G₁, G₂, B₁, B₂ und M₁, Deoxynivalenol "DON", Fumonisine B₁, B₂ und B₃, Ochratoxin A und Zearalenon nachzuweisen.

5. Die Multi-Analyt-Säule von irgendeinem der Ansprüche 1 bis 4, wobei das Harz eine Partikelgröße von 45 bis 165 µm aufweist, und/oder
wobei das Harz 5 mg Antikörper pro ml Harz umfaßt.

6. Die Multi-Analyt-Säule von irgendeinem der Ansprüche 1 bis 5, wobei ein eine Toxin-spezifische Affinität aufweisendes Harz in eine Säule geschichtet wird, nach und nach gefolgt vom Schichten eines anderen, eine andere Toxin-spezifische Affinität aufweisenden Harzes in die Säule, bis all das Harz in der Säule ist, oder
wobei die verschiedene Toxin-spezifische Affinität aufweisenden Harze gemischt und in eine Säule eingebracht werden.

7. Ein Verfahren zum Analysieren einer einzelnen flüssigen Probe bezüglich Aflatoxin, Deoxynivalenol, Fumonisin, Ochratoxin und Zearalenon, wobei das Verfahren umfaßt:
Bereitstellen einer wie in irgendeinem der Ansprüche 1 bis 6 angegebenen Multi-Analyt-Säule;
Beladen der Säule mit einer vorher festgelegten Menge einer flüssigen Probe, von der vermutet wird, daß sie eines oder mehrere der aus Aflatoxin, Deoxynivalenol, Fumonisin, Ochratoxin und Zearalenon ausgewählten Toxine umfaßt;
Binden der Toxine an die Antikörper auf der Säule;
anschließend Eluieren jedes der Toxine in Eluens; und
Analysieren des Eluens bezüglich des Vorhandenseins jedes Toxins.

8. Das Verfahren gemäß Anspruch 7, wobei Toxine unter Verwendung eines Wasser-basierten oder Wasser-kompatiblen Lösungsmittels von einem Lebensmittel extrahiert werden.

9. Das Verfahren gemäß Anspruch 8, wobei das Lösungsmittel Wasser:Methanol, Wasser:Acetonitril, Ethanol, Wasser:Ethanol, eine Salzlösung oder eine Pufferlösung ist.

10. Das Verfahren gemäß Anspruch 7, wobei die flüssige Probe irgendeine aus der aus einem Lebensmittelextrakt, einem Getreideextrakt und einem alkoholischen Getränk bestehenden Gruppe umfaßt.

11. Eine Verwendung einer wie in irgendeinem der Ansprüche 1 bis 6 angegebenen Multi-Analyt-Säule zum Analysieren eines Produkts oder einer Komponente eines Lebensmittels, zum Analysieren eines alkoholischen Getränks, zum Analysieren Pilzinfizierter Getreide und Früchte oder zum Analysieren von Malzgetränken und Weinen.

## Revendications

1. Colonne multi-analyte comprenant au moins une unité de résine ayant une affinité spécifique pour l'ochratoxine et, pour chaque unité de résine ayant une affinité spécifique pour l'ochratoxine, la colonne contient, en outre, 0,95 à 1,05 unités de résine contenant un anticorps ayant une spécificité pour la zéaralénone, 1,9 à 2,1 unités de résine contenant un anticorps ayant une spécificité pour l'aflatoxine, 2,35 à 2,65 unités de résine contenant un anticorps ayant une spécificité pour la fumonisine et 4,7 à 5,3 unités de résine contenant un anticorps ayant une spécificité pour le désoxynivalénol, une unité de résine désignant la quantité de résine contenant un anticorps qui se liera à 50 ng d'aflatoxine, 500 ng de désoxynivalénol, 3300 ng de fumonisine, 50 ng d'ochratoxine ou 1140 ng de zéaralénone, respectivement.

2. Colonne multi-analyte selon la revendication 1, dans laquelle la colonne est structurée et agencée pour récupérer au moins 60 %, de préférence, au moins 70 % d'une toxine dans un échantillon de 10 ml en cours d'analyse, ladite toxine étant choisie dans le groupe constitué par l'ochratoxine, la zéaralénone, l'aflatoxine, la fumonisine et le désoxynivalénol.

3. Colonne multi-analyte selon les revendications 1 ou 2, dans laquelle la colonne est structurée et agencée pour avoir un débit de colonne d'au moins 3 ml par minute, ou
dans laquelle un débit de fluide d'essai dans la colonne est de 1 à 2 gouttes par seconde.

4. Colonne multi-analyte selon l'une quelconque des revendications 1 à 3, dans laquelle la colonne est capable d'analyser un échantillon pour détecter chacune des aflatoxines G₁, G₂, B₁, B₂ et M₁, le désoxynivalénol "DON", les fumonisines B₁, B₂ et B₃, l'ochratoxine A et la zéaralénone.

5. Colonne multi-analyte selon l'une quelconque des revendications 1 à 4, dans laquelle la résine a une taille de particules de 45 à 165 µm, et/ou
dans laquelle la résine comprend 5 mg d'anticorps par ml de résine.

6. Colonne multi-analyte selon l'une quelconque des revendications 1 à 5, dans laquelle une résine ayant une affinité spécifique pour une toxine est placée dans une colonne, suivie successivement, par le placement dans la colonne d'une autre résine ayant une affinité spécifique pour une toxine différente jusqu'à ce que toute la résine soit dans la colonne, ou
dans laquelle les résines ayant une affinité spécifique pour une toxine différente sont mélangées et placées dans une colonne.

7. Procédé d'analyse d'un seul échantillon liquide pour l'aflatoxine, le désoxynivalénol, la fumonisine, l'ochratoxine et la zéaralénone, le procédé comprenant les étapes suivantes :
préparer une colonne multi-analyte comme indiqué dans l'une quelconque des revendications 1 à 6 ;
charger la colonne avec une quantité prédéterminée d'un échantillon liquide suspecté de contenir une ou plusieurs toxines choisies parmi l'aflatoxine, le désoxynivalénol, la fumonisine, l'ochratoxine et la zéaralénone ;
lier les toxines aux anticorps sur la colonne ;
ensuite, éluer chacune des toxines dans l'éluant ; et analyser l'éluant pour détecter la présence de chaque toxine.

8. Procédé selon la revendication 7, dans lequel les toxines sont extraites d'un aliment en utilisant un solvant à base d'eau ou compatible avec l'eau.

9. Procédé selon la revendication 8, dans lequel le solvant est l'eau/méthanol, l'eau/acétonitrile, l'éthanol, l'eau/éthanol, une solution saline ou une solution tampon.

10. Procédé selon la revendication 7, dans lequel l'échantillon liquide comprend l'un quelconque des échantillons liquides du groupe constitué par un extrait alimentaire, un extrait de grain, et une boisson alcoolisée.

11. Utilisation d'une colonne multi-analyte telle que décrite dans l'une quelconque des revendications 1 à 6 pour analyser un produit ou un ingrédient alimentaire, pour analyser une boisson alcoolisée, pour analyser des grains et des fruits infectés par des champignons, ou pour analyser des boissons maltées et des vins.
